# EUROPEAN PATENT APPLICATION

(11) **EP 1 197 553 A1**
(43) Date of publication of application: **17.04.2002**
(21) Application number: 00121394.1
(22) Date of filing: 12.10.2000
(51) Int. Cl.: C12N 15/11, A61K 31/70, C07H 21/04, A61K 47/48, A61P 17/00, A61P 29/00, A61P 35/00

(54) **Antisense nucleic acid against alphaV integrin**

(71) Applicant: A3D GmbH, Antisense Design & Drug Development, 69123 Heidelberg (DE)
(72) Inventor: Kronenwett, Ralf, Dr., 40237 Düsseldorf (DE); Gräf, Thorsten, 40591 Düsseldorf (DE); Haas, Rainer, Prof. Dr., 40237 Düsseldorf (DE); Nedbal, Wolfgang, Dr., 69121 Heidelberg (DE)
(74) Representative: Müller-Boré & Partner Patentanwälte

(57) **Abstract**

The present invention relates to nucleic acid constructs for inhibiting the expression of αV integrins in mammalian cells, as well as to pharmaceutical compositions containing said antisense constructs.

### Annex to the application documents - subsequently filed sequences listing

SEQUENCE LISTING

<110> A3D GmbH, Antisense Design & Drug Development

<120> Antisense nucleic acid against alphaV integrin

<130> A 3294EU

<140> 00 121 394.1
<141> 2000-10-12

<160> 7

<170> PatentIn Ver. 2.1

<210> 1
<211> 20
<212> DNA
<213> Artificial Sequence

<220>
<223> Description of Artificial Sequence: Antisense ODN
      directed against alphaV integrin chain

<400> 1
gaaaagccat cgccgaagtg                                              20

<210> 2
<211> 20
<212> DNA
<213> Artificial Sequence

<220>
<223> Description of Artificial Sequence: Antisense ODN
      directed against alphaV integrin chain

<400> 2
ttggaaatat aaggaaaagt                                              20

<210> 3
<211> 20
<212> DNA
<213> Artificial Sequence

<220>
<223> Description of Artificial Sequence: Antisense ODN

directed against alphaV integrin chain

<400> 3
tataggttgg cttgaaggat                                                    20



<210> 4
<211> 20
<212> DNA
<213> Artificial Sequence

<220>
<223> Description of Artificial Sequence: Antisense ODN
      directed against alphaV integrin chain

<400> 4
gcggaaaagc catcgccgaa                                                    20



<210> 5
<211> 20
<212> DNA
<213> Artificial Sequence

<220>
<223> Description of Artificial Sequence: Antisense ODN
      directed against alphaV integrin chain

<400> 5
aataaaatgc tttttttata                                                    20



<210> 6
<211> 20
<212> DNA
<213> Artificial Sequence

<220>
<223> Description of Artificial Sequence: Antisense ODN
      directed against alphaV integrin chain

<400> 6
tattttaata aaatgctttt                                                    20



<210> 7
<211> 20
<212> DNA

<213> Artificial Sequence

<220>
<223> Description of Artificial Sequence: Antisense ODN
      directed against alphaV integrin chain

<400> 7
tggaaatata aggaaaagta                                                    20

## Description

The present invention relates to nucleic acid constructs for inhibiting the expression of αV integrins in mammalian cells, as well as to pharmaceutical compositions containing said antisense constructs.

Integrins are a family of cell surface proteins that facilitate cell adhesion and play a major role in a number of biological processes including platelet aggregation, immune fuctions, tissue repair, cell proliferation, tumor invasion, inflammation, and inherited diseases. They are composed of two subunits α and β. So far, more than 11 α subunits and more than 6 β subunits are known. The subunit isotype αV is part of a number of α-β heterodimers including β1, β3, β5, β6 and β8. In the following, the integrin αVβ3 will be described as an example in more detail.

The integrin αVβ3 (also known as vitronectin receptor) is a heterodimeric protein which is expressed on the surface of various cell types and which functions as an adhesion molecule. Integrin αVβ3 consists of two subunits: The β3 chain which is present in all integrins of the β3-type and the specific αV chain. Integrin αVβ3 serves as a receptor for several extracellular matrix proteins such as vitronectin, fibronectin, laminin and collagen. This adhesion molecule is found, e.g. on activated endothelial and vascular muscle cells. Furthermore, this receptor is expressed on activated leucocytes, macrophages, osteoclasts and on cells of several types of tumors such as metastatic malignant melanoma, glioblastoma, urothelial carcinoma, breast cancer, ovarian carcinoma, prostatic carcinoma, astrocytoma, lung cancer, colon carcinoma, pancreatic carcinoma and osteosarcoma. αV integrins, in particular integrin αVβ3, play a central role in angiogenesis since their upregulation on endothelial cells promotes migration and sustains survival of vascular cells in angiogenic blood vessels. Neoangiogenesis is, in turn, important for many physiological processes such as wound healing, but also for pathological disorders such as growth of solid tumors or hematological neoplasias, restenosis after angioplasty, stenosis of vein bypasses, chronic inflammatory diseases and diabetic retinopathy. Therefore, the inhibition of neoangiogenesis represents a promising approach for the treatment of the above-mentioned pathological conditions.

So far, cancer diseases were treated by surgery, radiation therapy or chemotherapy. Recently, treatments using growth factors or immunological therapeutic approaches have been developed. The success thereof is highly dependent on the type of disease. However, concerning restenosis after angioplasty there are no successful therapeutic regimens available. Recent approaches using gene therapy are based on the local production of vasodilators. However, the effectiveness of such approaches remains to be shown. Chronic inflammatory diseases are commonly treated with non-steroidal antiphlogistics and corticosteroids. As a molecular therapeutic approach, antisense oligonucleotides directed against the adhesion molecule ICAM-1 have been developed recently (Yacyshyn et al., 1998, Gastroenterology 114: 1133-1142).

Recently, several substances for the inhibition of neoangiogenesis have been evaluated in clinical trials. Among them are interferons, angiogenesis-inhibiting cytokins, various substances which bind proangiogenic factors, substances which interfere with the matrix-endothelial interaction and thalidomide (reviewed in Gradishar, 1997, Invest. New Drugs 15: 49-59; Kuiper et al., 1998, Pharmacolog. Res. 37: 1-16).

Furthermore, a first specific inhibitor of integrin αVβ3 has been developed recently. This inhibitor is based on a peptide which binds to the αV subunit, thereby blocking the receptor. The inhibitor is suggested to be used in the treatment of malignant tumors (malignant melanoma, colon carcinoma, neuroblastoma); cf. Lode et al., 1999, Proc. Natl. Acad. Sci. USA 96: 1591-1596.

All of the above-mentioned therapeutic approaches show one or more disadvantages. For example, chemotherapy and radiation therapy are unspecific and consequently have severe side effects. Furthermore, most tumors become resistant against chemotherapeutics. Corticosteroids and non-steroidal antiphlogistics also show severe side-effects. The specific blockage of integrin αVβ3 by monoclonal antibodies or peptides may lead to immunological reactions against these substances. Furthermore, the preparation of antibodies with the required quality and quantity is difficult and expensive. Moreover, besides their possible immunogenicity, peptides are often unstable in serum.

Accordingly, the technical problem underlying the present invention is to provide a novel system for the specific inhibition of the expression of αV integrins, e.g. in order to inhibit neoangiogenesis.

The solution to the above technical problem is achieved by providing the embodiments of the present invention as characterized in the claims.

In particular, the present invention provides a nucleic acid, e.g. an antisense nucleic acid, comprising a nucleotide sequence which is capable of binding to a transcription product of the gene coding for the αV integrin chain thereby inhibiting the expression of αV integrins in mammalian cells by at least 50%.

Preferably, the nucleic acid according to the present invention is capable of inhibiting the expression of the αV integrins selected from a group including αVβ1, αVβ3, αVβ5, αVβ6 and αVβ8. A more preferred example is the inhibition of expression of integrin αVβ3.

The terms "nucleic acid" and "nucleotide sequence" refer to endogenously expressed, semisynthetic or chemically modified nucleic acid molecules of deoxyribonucleotides and/or ribonucleotides.

The term "transcriptional product" comprises all nucleic acid molecules which are primarily produced through transcription of the corresponding gene by the transcription machinery of the cell, or which are processed to mature mRNA by the splice apparatus and the further enzymatic systems for the formation of the 5'-cap and the 3'-poly-A⁺ tail.

The term "binding" means, that the nucleic acid according to the present invention and the transcription product of the gene coding the αV integrin chain are capable of interacting specifically which may comprise the formation of covalent chemical bonds, electrostatic interactions based on salt bridges, or bonds based on hydrogen bridges and/or hydrophobic interactions as well as binding due to base stacking. In particular, hydrogen bonds and hydrophobic interactions are the driving forces for binding through hybridization between the nucleic acid according to the present invention and the transcription product of the gene coding for the αV integrin chain. A hybridization between the above-defined nucleic acid and the αV integrin transcription product may, for example, occur in case at least a part of the nucleotide sequence of the nucleic acid according to the present invention is, at least in part, complementary to at least a part of the mRNA coding for the αV integrin chain.

In a preferred embodiment of the present invention, the mammalian cells are human cells which are preferably selected from endothelial cells, vascular muscle cells, activated leukocytes, macrophages, osteoclasts and tumor cells such as cells from the metastating malignant melanoma, glioblastoma, urothelial carcinoma, breast cancer, ovarian carcinoma, prostatic carcinoma, astrocytoma, lung cancer, colon carcinoma, pancreatic carcinoma and osteosarcoma.

The nucleic acid according to the present invention may contain deoxyribonucleotides and/or ribonucleotides. Preferably, it contains modified nucleotides such as phosphorothiate nucleotides, aminonucleotides, [α-S]-trisphosphate nucleotides, pseudouridine nucleotides and nucleotides having bases containing chemical modifications such as methyl, acetyl or isopentenyl groups. Furthermore, the nucleic acid may be modified by gap-mer technology or may be a locked nucleic acid (LNA).

According to a further preferred embodiment, the nucleic acid according to the present invention is an antisense nucleic acid, wherein the nucleotide sequence contains at least one of the following sequences: 5'-GAAAAGCCATCGCCGAAGTG-3' (SEQ ID NO 1), 5'-TTGGAAATATAAGGAAAAGT-3' (SEQ ID NO 2), 5'-TATAGGTTGGCTTGAAGGAT-3' (SEQ ID NO 3), 5'-GCGGAAAAGCCATCGCCGAA-3' (SEQ ID NO 4), 5'-AATAAAATGCTTTTTTTATA-3' (SEQ ID NO 5), 5'-TATTTTAATAAAATGCTTTT-3' (SEQ ID NO 6), or

5'-TGGAAATATAAGGAAAAGTA-3' (SEQ ID NO 7).

The term "antisense nucleic acid" means that at least a part of the nucleotide sequence of the nucleic acid is at least in part complementary to at least a part of a nucleotide sequence of the transcription product of the gene coding for the αV integrin chain.

A further embodiment of the present invention relates to a vector comprising the above-defined nucleic acid. The term "vector" refers to a DNA and/or RNA replicon that can be used for the amplification of one or more heterologuous or homologuous nucleotide sequences. The vector according to the present invention may contain regulatory elements known in the art. In particular useful in the context of the present invention are DNA or RNA vectors which upon transcription provide the nucleic acid according to the present invention. An example is a DNA vector encoding the nucleic acid according to the present invention downstream of the 3'-end of a suitable promoter.

A further embodiment of the present invention relates to a host organism containing the nucleic acid or the vector as defined above. The term "host organism" according to the present invention relates to any suitable prokaryotic or eukaryotic cell or may be a virus.

A further embodiment of the present invention relates to a method for the production of the above-defined nucleic acid or the vector, comprising cultivating the above-defined host organism under suitable conditions such as culture medium temperature etc. Preferably, the method further comprises isolating the desired products from the culture (cells and/or culture medium) according to procedures known in the art.

According to a further embodiment, the present invention provides a pharmaceutical composition containing the above-defined nucleic acid or the vector, optionally in combination with a pharmaceutically acceptable carrier and/or diluent.

A preferred embodiment of the pharmaceutical composition according to the present invention relates to the treatment of pathological disorders by modulation of cell adhesion. More preferably, the pharmaceutical composition according to the present invention affects platelet aggregation, immune functions, tissue repair, cell proliferation, tumor invasion, inflammation and inherited diseases. In case of the inhibition of the expression of integrin αVβ3, the pharmaceutical composition according to the present invention is particularly useful for the treatment of pathological disorders by inhibition of neoangiogenesis.

Examples of disorders which can be treated with the pharmaceutical composition according to the present invention comprise the group of cancer diseases such as the growth of solid tumors such as metastating malignant melanoma, glioblastoma, urothelial carcinoma, breast cancer, ovarian carcinoma, prostatic carcinoma, astrocytoma, lung cancer, colon carcinoma, pancreatic carcinoma and osteosarcoma or hematologic neoplasias such as multiple myeloma, acute and chronic leukemias, myelodysplastic syndrome and malignant lymphomas, restenosis after angioplasty, stenosis of vein bypasses, chronic inflammatory diseases such as Crohn's disease, ulcerative colitis, rheumatiod arthritis, ocular neovascular diseases such as diabetic retinopathy, disorders associated with excessive bone resorption such as osteoporosis, the above-mentioned multiple myeloma and bone metastasis, disorders of mammalian oral cavity which are related to inflammatory processes, e.g. concerning the gingival mucosa, such as gingivitis and peritonitis, and inflammatory skin disorders such as psoriasis, allergies, neurodermitis and akne.

A further embodiment of the present invention relates to a cosmetological composition containing the above-defined nucleic acid or the vector, optionally in combination with a cosmetologically acceptable carrier and/or diluent.

An application of the nucleic acid, the vector, the host organism or the pharmaceutical composition according to the present invention relates, for example, to a method for the inhibition of the expression of αV integrins such as integrin αVβ3 in mammals comprising introducing the nucleic acid, the vector, the host organism or the pharmaceutical composition according to the present invention in the mammalian organism. The method may be used, e.g. for the treatment of pathological disorders by modulation of cell adhesion, e.g. in order to inhibit neoangiogenesis, preferably for the treatment of the diseases as mentioned above.

The term "mammalian organism" comprises any type of single cells derived from mammals such as culture cells, as well as the mammal *per se.* Preferred mammalian organisms include man, dog, canine, cat, cow, pig and horse.

The term "introducing" into a mammalian organism may, e.g. in the case of single cells such as culture cells of mammalian cell lines, comprise a chemical transfection such as the Ca-phoshphate procedure or lipofection, or an electroporation, in the case of the whole animal or the human a subcutaneous, percutaneous, intramuscular, oral, intratracheal, peritoneal or intravenous and intraarterial administration of the nucleic acid, the vector, the host organism or the pharmaceutical composition according to the present invention. An intravenous or intraarterial administration may comprise, for example, a bolus injection and/or a continuous infusion.

Another embodiment of the present invention relates to a diagnostic kit containing the above-defined nucleic acid and/or the vector and/or the host organism. The diagnostic kit according to the present invention may, e.g. be used for the detection of expression levels of αV integrins. For example, the above-defined nucleic acid contained in the diagnostic kit according to the present invention may be used for PCR for the detection of αV integrin mRNA.

The figures show:
- Fig. 1: is a graphical representation showing the relative αV expression after stimulation with phorbol-12-myristate-13-acetate (PMA) in human umbilical vein endothelial cells (HUVEC) which have been transfected with antisense oligodeoxyribonucleotides (ODN) according to the present invention (filled bars) or with control ODN (unfilled bars). The basis-αV-expression of unstimulated cells was set to 0% and the expression following stimulation by PMA was set to 100%.
- Fig. 2: is a graphical representation showing the relative proliferation rate of HUVEC cells transfected with antisense ODN according to the present invention (filled bars) or with control ODN (unfilled bar). The proliferation rate of untransfected stimulated cells was set to 100%.
- Fig. 3: is a graphical representation showing the annexin binding as a measure of apoptosis rate (expressed as mean fluorescence intensity) of HUVEC cells transfected with an ODN according to the present invention (av32, filled bar) or with a control ODN (avSC32, unfilled bar).

The present invention is further illustrated by the following non-limiting example.

### EXAMPLE

### Computer-aided selection of target sequences

The secondary structures of the αV mRNA were calculated with the computer program mfold 2.0 of the Heidelberg Unix Sequence Analysis Resources (HUSAR). The secondary structures were systematically analysed by the method of Patzel et al. (Nucl. Acids Res. 27: 4823-4334, 1999). On the basis of these analyses, suitable local target sequences were identified.

### Antisense oligonucleotides

Selected antisense ODN and control ODN were all phosphorothioate-modified and were synthesized by a commercial supplier.

The antisense oligonucleotides which showed the best performance in the inhibition of αV expression had the following sequences:

av 32:     5'-GAAAAGCCATCGCCGAAGTG-3'     (pos:32-51, SEQ ID NO 1)

av 4175: 5'-TTGGAAATATAAGGAAAAGT-3' (pos.4175-4194, SEQ ID NO 2)

av 3880: 5'-TATAGGTTGGCTTGAAGGAT-3' (pos. 3880-3899, SEQ ID NO 3)

av 35: 5'-GCGGAAAAGCCATCGCCGAA-3' (pos. 35-54, SEQ ID NO 4)

av 4044: 5'-AATAAAATGCTTTTTTTATA-3' (pos. 4044-4063, SEQ ID NO 5)

av 4050: 5'-TATTTTAATAAAATGCTTTT-3' (pos. 4050-4069, SEQ ID NO 6 )

av 4174:  5'-TGGAAATATAAGGAAAAGTA-3'   (pos. 4174-4193, SEQ ID NO 7)

The positions indicated above refer to the αV cDNA sequence as published by Suzuki et al. (J. Biol. Chem. 262:14080-14085, 1987).

### Transfection

The activity of antisense ODN was tested on human umbilical vein endothelial cells (HUVEC). The oligonucleotides (0,05 µM final concentration) were transfected in HUVEC using lipofectin as carrier. The cells were stimulated with PMA after 8 hours. After further 48 hours, the expression of the αV integrin chain, the proliferation and the apoptosis rate were studied.

### Functional studies

Expression of the αV integrin chain was determined by flowcytometry. Using the mean fluorescence intensity of the cells in each sample, the inhibition of the PMA-induced expression of the αV integrin chain by the ODN was calculated.

The proliferation after transfection with ODN was determined by photometrical measurement of the BrdU incorporation. The apoptosis rate was determined by flowcytometry by the binding of annexin.

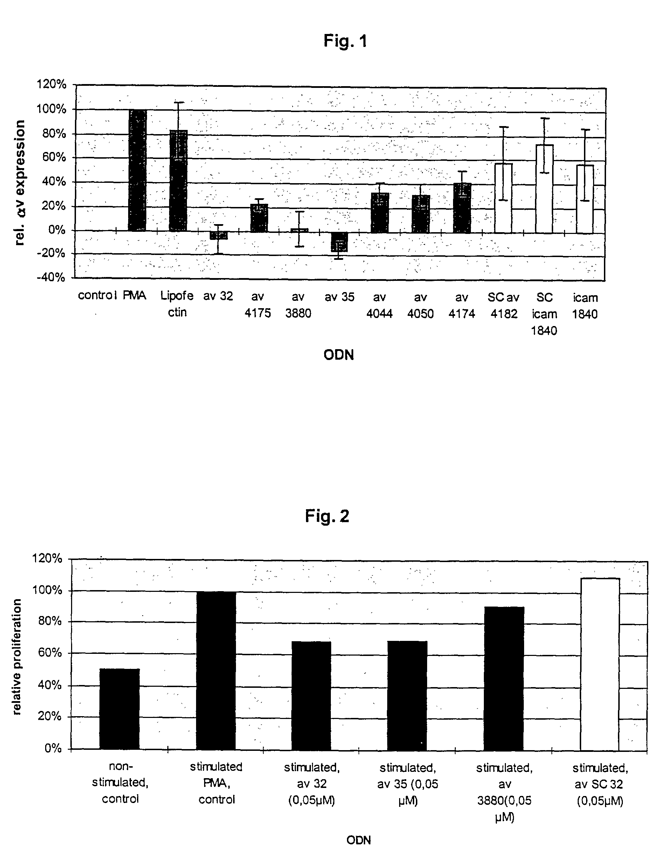

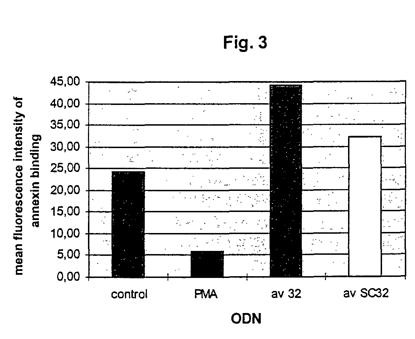

As assessed by flowcytometry already after 48 hours following transfection, 3 out of 7 antisense ODN inhibited the upregulation of αV completely at an ODN concentration of 0,05 µM. Furthermore, all ODN according to the present invention surprisingly inhibit the upregulation of αV by more than 50% at this low ODN concentration (see Fig. 1). The inhibition of αV expression was furthermore associated with an inhibition of proliferation of HUVEC cells by 32% (Fig. 2) and led to a 5-fold increase in the apoptosis rate of antisense-transfected cells in comparison to control cells (Fig. 3).

In addition, pretreatment of HUVEC cells with an αV-directed antisense oligonucleotide inhibited the formation of tubular structures in an endothelial cell tube assay using Matrigel-coated cell culture plates.

In conclusion, antisense sequences were identified which, after transfection in form of DNA oligonucleotides, lead to an inhibition of PMA-induced αV-expression in endothelial cells. The inhibition of αV-expression was associated with a decreased proliferation of the endothelial cells and an increased apoptosis rate. Therefore, the present invention provides for the first time a highly specific inhibitor of αV-expression, e.g. in endothelial cells, on the basis of antisense oligonucleotides which do not act by interfering with the extracellular protein-protein interaction but which act intracellularly by downregulation of the expression of the receptor. In comparison to other αV-antagonists the nucleic acid inhibitors of the present invention can be prepared easily in large amounts and in good manufacturing practice (GMP) quality by simple chemical synthesis.

The antisense-based inhibitors of the present invention can be used successfully in inhibition of neoangiogenesis, e.g. for the treatment or prevention of restenosis following angioplasty or for the treatment of malignant tumors or hematological malignancies such as multiple myeloma.

## Claims

1. A nucleic acid comprising a nucleotide sequence which is capable of binding to a transcription product of the gene coding for the αV integrin chain thereby inhibiting the expression of αV integrins in mammalian cells by at least 50%.

2. The nucleic acid according to claim 1, wherein the αV integrin is selected from the group consisting of integrin αVβ1, αVβ3, αVβ5, αVβ6 and αVβ8.

3. The nucleic acid according to claim 2, wherein the αV integrin is integrin αVβ3.

4. The nucleic acid according to any one of claims 1 to 3, wherein the mammalian cells are human cells.

5. The nucleic acid according to claim 4, wherein the human cells are selected from endothelial cells, vascular muscle cells, activated leukocytes, macrophages, osteoclasts and tumor cells.

6. The nucleic acid according to any one of claims 1 to 5 containing modified nucleotides.

7. The nucleic acid according to claim 6, wherein the modified nucleotides are phosphorothioate nucleotides.

8. The nucleic acid according to any one of claims 1 to 7, wherein the nucleotide sequence contains at least one of the sequences of SEQ ID NO 1 to 7.

9. A vector comprising the nucleic acid according to any one of claims 1 to 8.

10. A host organism containing the nucleic acid according to any one of claims 1 to 8 or the vector according to claim 9.

11. A method for the production of the nucleic acid according to any one of claims 1 to 8 or the vector according to claim 9, comprising cultivating the host organism according to claim 10.

12. A pharmaceutical composition containing the nucleic acid according to any one of claims 1 to 8 or the vector according to claim 9, optionally in combination with a pharmaceutically acceptable carrier and/or diluent.

13. The pharmaceutical composition according to claim 12 for the treatment of pathological disorders by modulation of cell adhesion.

14. The pharmaceutical composition according to claim 13, which affects platelet aggregation, immune functions, tissue repair, cell proliferation, tumor invasion, inflammation and inherited diseases.

15. The pharmaceutical composition according to claim 13 or 14, wherein the disorder is selected from the group of cancer diseases, restenosis after angioplasty, stenosis of vein bypass, chronic inflammatory diseases, ocular neovascular diseases, disorders associated with excessive bone resorption, disorders of mammalian oral cavity and inflammatory skin disorders.

16. A cosmetological composition containing the nucleic acid according to any one of claims 1 to 8 or the vector according to claim 9, optionally in combination with a cosmetologically acceptable carrier and/or diluent.

17. A diagnostic kit containing the nucleic acid according to any one of claims 1 to 8 and/or the vector according to claim 9 and/or the host organism according to claim 10.
